# EUROPEAN PATENT SPECIFICATION

(11) **EP 1 214 907 B1**
(45) Date of publication and mention of the grant of the patent: **28.06.2006**
(21) Application number: 01500284.3
(22) Date of filing: 11.12.2001
(51) Int. Cl.: A61B 5/16

(54) **Mental-state analysing device for mammals**
Vorrichtung zur Analyse des mentalen Zustandes von Säugetieren
Dispositif pour l'analyse de l'état mental de mammifères

(30) Priority: 13.12.2000 ES 200002975
(43) Date of publication of application: 19.06.2002
(73) Proprietor: Monagas Asensio, Pedro, 08211 Castellar Del Valles (Barcelona) (ES)
(72) Inventor: Monagas Asensio, Pedro, 08211 Castellar Del Valles (Barcelona) (ES)
(74) Representative: Ponti Sales, Adelaida

(56) References cited:
- WO-A-00/21048
- BE-A- 906 169
- US-A- 5 668 780
- US-A- 5 853 005
- PATENT ABSTRACTS OF JAPAN vol. 1998, no. 05, 30 April 1998 (1998-04-30) & JP 10 011674 A (NIPPON DENKI IDO TSUSHIN KK), 16 January 1998 (1998-01-16)
- PATENT ABSTRACTS OF JAPAN vol. 016, no. 086 (C-0916), 3 March 1992 (1992-03-03) & JP 03 272710 A (MATSUSHITA ELECTRIC WORKS LTD), 4 December 1991 (1991-12-04)

## Description

This invention relates to a mental-state analysing device for mammals, which can be used with any mammal, since a single device can be equipped with various standard patterns.

### BACKGROUND OF THE INVENTION

There currently exist in the market various appliances designed and configured to alert about the occurrence of extraneous factors which alter the well-being of babies and impaired persons.

For example, there exist ambient-temperature detectors, which activate when the temperature in the room undergoes sudden changes.

There also exist noise sensors, which activate when a baby cries, and likewise known are other widely disseminated appliances which act simply as wireless microphones or sound transmitters, which send the signal of the crying to a receiver which is usually in the possession of a responsible adult.

All these currently known devices have the disadvantage of activating for a single factor, and they in an any case indicate absolute values. In the case of temperature, for example, the devices indicate the degrees of temperature in the room in which the detector is situated.

The device disclosed in patent JP-10011674A detects temperature and perspiration values of infants together with ambient-temperature and ambient-sound values. Then, the measured values are transmitted to a CPU and an abnormality notification signal is selected and transmitted. Said device has the disadvantage of only detecting extraneous factors and therefore abnormal situations but not the mental-state of the infant and can only be used with infants.

US-5853005A discloses a device for monitoring biological activity of a living organism. Said device uses the acoustic characteristics of body functions such as heart rate, pulmonary function, respiration to diagnose medical conditions. Said device does not permit to determine the state of mind of any mammal.

As well, patent US-5668780A discloses a baby cry recogniser. It detects the sounds emitted by the baby and determines if said sound is a baby cry. It can only be used for babies.

The detection and alerting device disclosed in patent WO 00/21048 (corresponding to Spanish patent P9802190), from the same holder as this application, was designed in order to resolve this disadvantage.

This device intervenes in any situation which involves an alteration in the conditions classified as normal or usual in the surroundings of the baby, and is capable of acting for temperature, relative humidity, ambient noise, air speed and ambient light.

The device described in this patent is programmed on the basis of parameters determined by approximate tolerance values, so that when one or more of the conditions in the surroundings of the baby or of the disabled person are modified or altered, the corresponding sensor activates the alert signal of a warning device, which is transmitted to the receiver in the possession of a responsible adult.

This device is also fitted with a sensor sensitive to the crying of a baby or the groans emitted by a disabled person or an elderly person, and is programmed to identify different intervals, frequency or intensity of the crying or groans, in such a way that it is possible to interpret these parameters and determine the reason for the crying or groaning in function of its characteristics.

This device, which fulfils its function perfectly with children and elderly persons, cannot be applied to any mammal, however. This is due to its being limited as regards the entry of standards, since it has some fixed standards with no possibility of changing the references easily. Even if the references could be changed, they would always have to be associated with the hardware and could under no circumstances be changed by software.

### DESCRIPTION OF THE INVENTION

The analysing device of the invention manages to solve the above-mentioned disadvantages, while presenting other advantages which will be described below.

The mental-state analysing device of the invention for mammals includes means of sound reception, means of processing the sound received connected to a memory which stores some predetermined parameters, and means of alarm and/or display, in such a way that the sound is analysed through the processing means, is compared with the predetermined parameters stored in the memory and the means of alarm and/or display are activated to indicate the mental state of the mammal, and is characterised in that said memory in interchangeable and said processing means are a digital signal processor, which analyses the frequency and time parameters of the sound.

Preferably, said frequency and time parameters of the sound analysed by the digital signal processor include the frequency, intensity, shape of the envelope, rate of repetition, energy content, pulse duration, wave shape, magnitude, self-correlation and density of zero crossings.

Advantageously, said means of alarm and/or display are housed in a portable receiver, which will be in the possession of a responsible person.

With the analysing device of this invention it is possible to determine the states of mind of any mammal.

Thanks to the use of a digital signal processor it is possible to pick up, analyse, process and compare noises and sounds emitted by mammals with an associated symptomology, in order to discern and distinguish between different variants, with the advantage that many standard patterns can be available for different species with the same device.

For example, it can be used for farm animals (cows, sheep, etc.), zoo mammals (monkeys, cats, herbivores, pachyderms, etc.), marine mammals (cetaceans, etc.), household-pet mammals (cats, dogs, etc.) and even impaired persons, babies and elderly persons.

The states of mind that can be detected are, for example, hunger, sleepiness, boredom, pain, stress and others.

Depending on the mammal to which it is applied a suitable memory card will have to be placed in the analysing device of this invention.

To provide greater certainty that the right state of mind has been detected, the analysing device of this invention can also include means for confirming the mental state detected where the number of questions can vary depending on the mammal to which the analysing device of this invention is applied.

According to a preferred embodiment, said means of confirmation include at least one pair of pushbuttons, one for affirmation and the other for negation, so that the responses via the pushbuttons to a number of questions indicated on the display serve to confirm or otherwise deny the mental state detected.

Said confirmation means can also include an indetermination pushbutton, in the event that the person replying feels unable to answer the question shown on the display.

### BRIEF DESCRIPTION OF THE DRAWINGS

For a better understanding of all that has been set out some drawings are attached which show, schematically and solely by way of non-restrictive example, a practical case of embodiment.

Figure 1 is a block diagram showing the components of the analysing device of this invention;

Figure 2 is a schematic view of the means of confirmation which form part of the analysing device of this invention.

### DESCRIPTION OF A PREFERRED EMBODIMENT

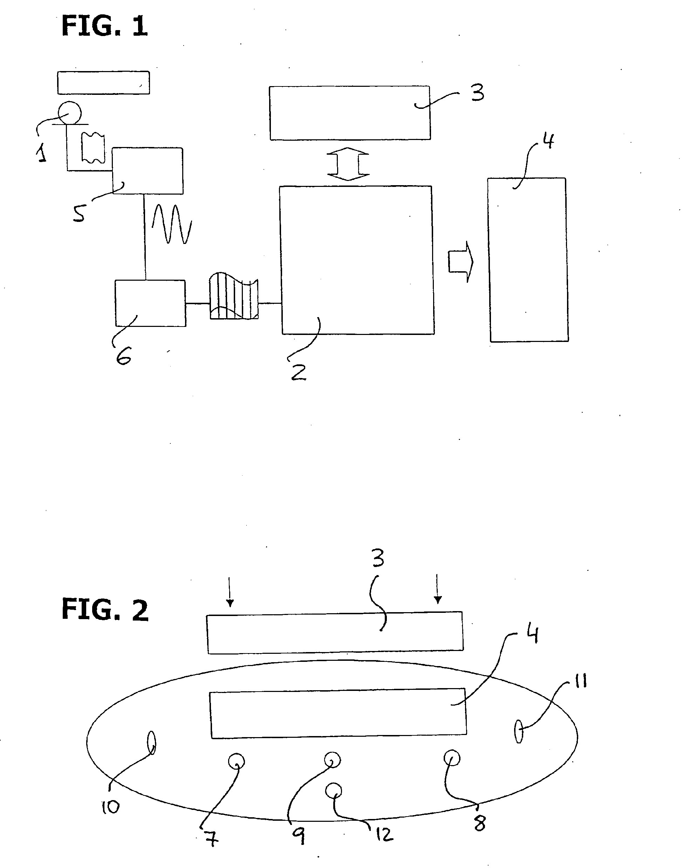

As Figure 1 shows, the analysing device of this invention includes a microphone 1 which is connected to a digital signal processor 2 through an analogical to digital converter 6 and a filter 5, which is provided with an amplifier. The digital signal processor 2 is in turn connected to a memory 3 and an alarm and/or display element 4.

It should be stated that the digital signal processors 2 applied in the analysing device of this invention are currently used in a wide variety of spheres and products, such as in mobile telephony, in audio and video appliances, in television and radio transmitters, etc.

The analysing device of this invention is used in the following manner.

The mammal with which the analysing device of this invention is going to be used should first be indicated. This indication can be carried out in different ways, such as by software or by inserting a memory card 3 (Figure 2) into the analysing device of this invention.

When a sound is detected through the microphone 1, the signal generated by the microphone is filtered and amplified in the filter 5 and in the amplifier, respectively. This analogical signal is then converted into a digital signal by means of the converter 6.

When the digital signal has been obtained, it is processed by the digital signal processor 2 by means of the corresponding software, by assigning different values to the different frequency and time parameters of the sound which are analysed.

Said parameters can clearly be very diverse and depend largely on the mammal whose state of mind is to be analysed; by way of non-restrictive example, however, they could be parameters such as the frequency, intensity, shape of the envelope, rate of repetition, energy content, pulse duration, wave shape, magnitude, self-correlation, density of zero crossings, etc.

The values obtained are compared with values that halve previously been entered in the interchangeable memory 3, in such a way that said comparison can determine the state of mind of the mammal.

When the state of mind has been determined by the sound detected, said information will be transmitted to a responsible person by the alarm and/or display means 4. The ways of representing said information can be very diverse, for example by emission of an acoustic, light or remote signal, or simply by indicating on a screen the state of mind detected. Preferably, however, said means of alarm and/or display are housed in a portable receiver.

In the case of the barking of a dog, for example, a succession of barks with a similar rate of repetition and with a similar energy at a certain frequency and a particular self-correlation would indicate excitability, or a howl with a certain repetition rate would indicate sadness.

If it is wished to check that the state of mind detected is correct the checking means shown in Figure 2 can be used.

These checking means include the screen 4 of the display means mentioned above, an on-off button 10, an affirmation pushbutton 7, a negation pushbutton 8 and an indetermination pushbutton 9. These pushbuttons are used to reply to the questions which appear on the screen 4.

Said confirmation means also include a pushbutton 11 for deleting the reply and a pushbutton 12 for going back to the previous question.

The number of questions can vary depending on the mammal to which the analysing device of this invention is applied.

For example, if it is used to check the state of mind which has been detected in a baby, it would ask if the baby shook its head, waved its arms, kicked its legs, clenched its fists, tried to suckle, sucked its fists, showed rigidity of the body, had wind and intestinal noises, raised its arms or cried.

On the basis of the replies to these questions a check can be made to see if the baby is hungry, bored, suffering pain, sleepiness or stress.

Furthermore, on the basis of the state of mind detected, the screen 4 or an adhesive sticker fixed onto the device can give advice about what to do. For example, if the baby was hungry it would advise giving it the feeding bottle or its mother's milk and checking whether it was thirsty, if it was bored then keeping it company, talking to it gently, taking it for a walk, etc.

Despite the fact that reference has been made to one specific embodiment of the invention, it will be clear to a person skilled in the art that the analysing device described lends itself to many variations and modifications without departing from the scope of protection defined in the attached claims.

## Claims

1. Mental-state analysing device for mammals, which includes means of sound reception (1), means of processing the sound received (2) connected to a memory (3) which stores some predetermined parameters, and means of alarm and/or display (4), in such a way that the sound is analysed through the processing means (2), is compared with the predetermined parameters stored in the memory (3) and the means of alarm and/or display (4) are activated to indicate the mental state of the mammal, **characterised in that** said memory is interchangeable and said processing means are a digital signal processor (2), which analyses the frequency and time parameters of the sound.

2. Analysing device as claimed in Claim 1, **characterised in that** said frequency and time parameters of the sound analysed by the digital signal processor (2) include the frequency, intensity, shape of the envelope, rate of repetition, energy content, pulse duration, wave shape, magnitude, self-correlation and density of zero crossings.

3. Analysing device as claimed in Claim 1, **characterised in that** said means of alarm and/or display are housed in a portable receiver.

4. Analysing device as claimed in Claim 1, **characterised in that** it also includes means for confirming the mental state detected.

5. Analysing device as claimed in Claim 4, **characterised in that** said means of confirmation include at least one pair of pushbuttons, one for affirmation (7) and the other for negation (8), so that the responses via said pushbuttons (7, 8) to a number of questions indicated on the display serve to confirm or otherwise deny the mental state detected.

6. Analysing device as claimed in Claim 5, **characterised in that** said confirmation means can also include an indetermination pushbutton (9), in the event that the person replying feels unable to answer the question shown on the display means (4).

## Patentansprüche

1. Vorrichtung zur Analyse des mentalen Zustandes von Säugetieren, welche eine Einrichtung zur Tonaufnahme (1), Einrichtungen zum Verarbeiten des empfangenen Tones (2), die mit einem Speicher (3) verbunden sind, der einige vorbestimmte Parameter speichert, und eine Einrichtung des Alarms und/oder der Anzeige (4) umfasst, derart, dass der Ton durch die Verarbeitungseinrichtungen (2) analysiert wird, mit den vorbestimmten Parametern, die in dem Speicher (3) gespeichert sind, verglichen wird, und die Einrichtungen des Alarms und/oder der Anzeige (4) aktiviert werden, um den mentalen Zustand des Säugetiers anzuzeigen, **dadurch gekennzeichnet, dass** der Speicher austauschbar ist, und dass die Verarbeitungseinrichtungen ein digitaler Signalsprozessor (2) ist, der die Frequenz- und die Zeitparameter des Tones analysiert.

2. Analysevorrichtung nach Anspruch 1, **dadurch gekennzeichnet, dass** die Frequenz- und die Zeitparameter des Tones, der durch den digitalen Signalprozessor (2) analysiert wird, die Frequenz, die Intensität, den Tonumfang, die Rate der Wiederholung, den Energiegehalt, die Pulsdauer, die Wellenform, die Magnitude, die Selbstkorrelation und Dichtheit von Null-Kreuzungen umfassen.

3. Analysevorrichtung nach Anspruch 1, **dadurch gekennzeichnet, dass** die Alarm- und/oder Anzeigeeinrichtungen in einem tragbaren Aufnahmegerät aufgenommen sind.

4. Analysevorrichtung nach Anspruch 1, **dadurch gekennzeichnet, dass** sie auch eine Einrichtung zur Bestätigung des erfassten mentalen Zustandes aufweist.

5. Analysevorrichtung nach Anspruch 4, **dadurch gekennzeichnet, dass** die Bestätigungseinrichtungen wenigstens ein Paar von Tasten umfassen, eine zur Bestätigung (7) und die andere zur Negation (8), so dass die Antwort über diese Tasten (7, 8) zu einer Mehrzahl von Fragen, die auf dem Bildschirm angegeben sind, dazu dienen, den erfassten mentalen Zustand zu bestätigen oder andererseits zu dementieren.

6. Analysevorrichtung nach Anspruch 5, **dadurch gekennzeichnet, dass** die Bestätigungseinrichtung auch eine Unbestimmtheit-Taste (9) umfassen kann, in dem Fall, in dem die Person sich nicht in der Lage fühlt, die Frage, die auf der Anzeigeeinrichtung (4) gezeigt ist, zu beantworten.

## Revendications

1. Dispositif pour analyser l'état mental pour des mammifères, qui comprend des moyens de réception sonore (1), des moyens de traitement du son reçu (2) raccordés à une mémoire (3) qui stocke certains paramètres prédéterminés, et des moyens d'alarme et/ou d'affichage (4), de telle sorte que le son est analysé au moyen des moyens de traitement (2), est comparé aux paramètres prédéterminés stockés dans la mémoire (3) et les moyens d'alarme et/ou d'affichage (4) sont activés pour indiquer l'état mental du mammifère, **caractérisé en ce que** ladite mémoire est interchangeable et lesdits moyens de traitement sont un processeur du signal numérique (2), qui analyse les paramètres de fréquence et de temps du son.

2. Dispositif d'analyse selon la revendication 1, **caractérisé en ce que** lesdits paramètres de fréquence et de temps du son analysé par le processeur du signal numérique (2) comprennent la fréquence, l'intensité, la forme de l'enveloppe, la cadence de répétition, le contenu énergétique, la durée d'impulsion, la forme d'onde, l'amplitude, l'auto-corrélation et la densité des passages par zéro.

3. Dispositif d'analyse selon la revendication 1, **caractérisé en ce que** lesdits moyens d'alarme et/ou d'affichage sont logés dans un récepteur portable.

4. Dispositif d'analyse selon la revendication 1, **caractérisé en ce qu'**il comprend aussi des moyens pour confirmer l'état mental détecté.

5. Dispositif d'analyse selon la revendication 4, **caractérisé en ce que** lesdits moyens de confirmation comprennent au moins une paire de boutons-poussoirs, l'un pour l'affirmation (7) et l'autre pour la négation (8), de sorte que les réponses au moyen desdits boutons-poussoirs (7, 8) à un certain nombre de questions indiquées sur l'afficheur servent à confirmer ou bien à infirmer l'état mental détecté.

6. Dispositif d'analyse selon la revendication 5, **caractérisé en ce que** lesdits moyens de confirmation peuvent aussi comprendre un bouton-poussoir d'indétermination (9), pour le cas où la personne qui répond se sentirait incapable de répondre à la question montrée sur les moyens d'affichage (4).
